# EUROPÄISCHE PATENTSCHRIFT

(11) **EP 3 092 481 B1**
(45) Veröffentlichungstag und Bekanntmachung des Hinweises auf die Patenterteilung: **25.07.2018**
(21) Anmeldenummer: 14809793.4
(22) Anmeldetag: 03.12.2014
(51) Int. Cl.: G01N 27/406, G01N 27/417

(54) **VERFAHREN UND VORRICHTUNG ZUR ÜBERWACHUNG DER FETTGAS-MESSFÄHIGKEIT EINER ABGAS-SONDE**
METHOD AND DEVICE FOR MONITORING THE CAPABILITY OF AN EXHAUST GAS PROBE TO MEASURE A FUEL RICH MIXTURE
PROCÉDÉ ET DISPOSITIF DE SURVEILLANCE DE LA CAPACITÉ DE MESURE D'UNE SONDE DE GAZ D'ÉCHAPPEMENT EN PRÉSENCE D'UN MÉLANGE RICHE

(30) Priorität: 07.01.2014 DE 102014200063
(43) Veröffentlichungstag der Anmeldung: 16.11.2016
(73) Patentinhaber: Robert Bosch GmbH, 70442 Stuttgart (DE)
(72) Erfinder: REINHARDT, Goetz, 71032 Boeblingen (DE); BUCHHOLZ, Martin, 74321 Bietigheim-Bissingen (DE)
(86) Internationale Anmeldenummer: PCT/EP2014/076380
(87) Internationale Veröffentlichungsnummer: WO 2015/104102

(56) Entgegenhaltungen:
- DE-A1-102010 040 817
- DE-A1-102011 005 490

## Beschreibung

Die Erfindung betrifft ein Verfahren zur Überwachung einer Fettgas-Messfähigkeit einer Abgas-Sonde in einem Abgaskanal einer mager betriebenen Brennkraftmaschine, wobei in der Abgas-Sonde Abgas über eine Diffusionsbarriere von dem Abgaskanal in einen Messhohlraum der Abgas-Sonde diffundiert und wobei über eine Pumpzelle mit einer inneren Pumpelektrode und einer zweiten Pumpelektrode durch Anlegen einer Pumpspannung zwischen den Elektroden Sauerstoff entsprechend eines fließenden Pumpstroms in den oder aus dem Messhohlraum gepumpt wird.

Die Erfindung betrifft weiterhin eine Vorrichtung zur Überwachung einer Fettgas-Messfähigkeit einer Abgas-Sonde in einem Abgaskanal einer mager betriebenen Brennkraftmaschine, wobei ein Messhohlraum der Abgas-Sonde über eine Diffusionsbarriere mit dem Abgaskanal verbunden ist, wobei eine Pumpzelle mit einer inneren Pumpelektrode und einer zweiten Pumpelektrode zum Pumpen von Sauerstoff in oder aus dem Messhohlraum vorgesehen ist, wobei zum Auspumpen von Sauerstoff aus dem Messhohlraum ein positiver und zum Einpumpen ein negativer Pumpstrom zwischen den Elektroden vorgesehen ist und wobei der Abgas-Sonde eine Steuereinheit zugeordnet ist.

Abgas-Sonden werden beispielsweise in Form von Lambda-Sonden im Abgaskanal von Brennkraftmaschinen zur Bestimmung der Zusammensetzung eines der Brennkraftmaschine zugeführten Luft-Kraftstoff-Gemischs verwendet. Gemäß gesetzlicher Vorschriften müssen die Abgas-Sonden im Betrieb auf korrekte Funktion, auf eine so genannte "Messfähigkeit", überprüft werden. Hierzu kann ein Gas-Gemisch bekannter Zusammensetzung der Abgas-Sonde zugeführt werden und das Ausgangssignal der Abgas-Sonde bewertet werden. Bei der Überprüfung einer Lambda-Sonde auf korrekte Funktion bei einem "fetten" Luft-Kraftstoff-Verhältnis mit einem Lambdawert < 1, der Überprüfung der so genannten "Fettmessfähigkeit", müsste der Lambda-Sonde somit ein solches Gemisch zugeführt werden. Insbesondere bei Dieselmotoren, die im normalen Betrieb im Bereich eines mageren Abgases betrieben werden, führt ein Fettbetrieb für eine Diagnose zu einer erhöhten Emission von Schadstoffen sowie zu einem zusätzlichem Kraftstoff-Verbrauch und wird deshalb vorzugsweise vermieden. Sonstige Möglichkeiten, eine fette Gas-Zusammensetzung an der Lambda-Sonde bereitzustellen, ohne einen Eingriff in den Motorbetrieb vorzunehmen, sind aus vielfachen Gründen nicht realisierbar.

Eine zuverlässige Diagnose auf anderem Weg ist allerdings auch mit Nachteilen behaftet. Zur Bestimmung des Lambdawerts wird der Pumpstrom der Lambda-Sonde verwendet. Der Pumpstrom ist bei magerem Abgas positiv, bei fettem Abgas negativ. Die Transportprozesse innerhalb der Lambdasonde bei magerem und fettem Abgas unterscheiden sich erheblich hinsichtlich Stromrichtung, Elektrodenreaktion und Gasen an den Elektroden. Für eine realitätsnahe Diagnose müssen diese Bedingungen daher, soweit zur Diagnose erforderlich, bereitgestellt werden.

Abgas-Sonden können als Breitband-Lambdasonden, Lambda-Sprungsonden, zweizellige Breitband-Lambdasonden oder als einzellige Grenzstromsonden, aber auch beispielsweise als NOₓ-Sensor ausgeführt sein.

Die DE 10 2011 005 490 A1 offenbart ein Verfahren zum Betrieb eines Sensorelements zur Erfassung mindestens einer Eigenschaft eines Gases in einem Messgasraum, wobei das Sensorelement mindestens eine Pumpzelle mit mindestens zwei Elektroden und mindestens einem die Elektroden verbindenden Festelektrolyten umfasst, wobei mindestens eine erste Elektrode der Pumpzelle mit Gas aus dem Messgasraum beaufschlagbar ist, wobei mindestens eine zweite Elektrode der Pumpzelle mit mindestens einem Referenzkanal in Verbindung steht, wobei bei dem Verfahren überprüft wird, ob ein Pumpstrom durch die Pumpzelle durch eine Beaufschlagung der ersten Elektrode mit Gas begrenzt wird oder ob der Pumpstrom durch den Referenzkanal begrenzt wird. In der Beschreibung der Erfindung wird auf die Möglichkeit einer Pumpspannungsumkehr zu Diagnosezwecken hingewiesen. In der Schrift wird jedoch nicht auf die Diagnose der Fettmessfähigkeit einer Abgas-Sonde eingegangen.

Die DE 10 2010 040 817 A1 beschreibt ein Verfahren zum Abgleich eines Sensorelements zur Erfassung mindestens einer Eigenschaft eines Gases in einem Messgasraum, insbesondere zur Bestimmung eines Anteils einer Gaskomponente, wobei das Sensorelement mindestens zwei Zellen mit jeweils mindestens zwei Elektroden und mindestens einem die Elektroden verbindenden Festelektrolyten aufweist, wobei mindestens eine erste Elektrode Bestandteil beider Zellen ist, wobei die erste Elektrode über mindestens eine Diffusionsbarriere mit Gas aus dem Messgasraum beaufschlagbar ist, wobei die Zellen mindestens eine erste Zelle und mindestens eine zweite Zelle umfassen, wobei mindestens eine von der ersten Elektrode aus durch eine zweite Zelle gepumpte Gaskomponente des Gases über die Diffusionsbarriere zumindest teilweise zur ersten Elektrode zurückführbar ist, wobei ein erster Pumpstrom durch die erste Zelle gemessen wird, wobei ein zweiter Pumpstrom durch die zweite Zelle gemessen wird, wobei aus dem ersten Pumpstrom und dem zweiten Pumpstrom auf mindestens eine Eigenschaft der Diffusionsbarriere geschlossen wird. In der Schrift wird offenbart, dass die Kennlinie einer Breitband-Lambdasonde während oder nach einer Produktion oder auch im Feldbetrieb, beispielsweise in einem Schubbetrieb eines Dieselmotors, abgeglichen werden kann. Dabei können beispielsweise mittels einer Pumpstromumkehr und/ oder einem Aufpumpen des Elektrodenhohlraums entsprechende Diagnoseverfahren und/ oder Regenerationsverfahren durchgeführt werden. Ein Verfahren zur Diagnose der Fettmessfähigkeit der Lambdasonde wird nicht offenbart.

Die Schrift DE 10 2009 060 172 A1 offenbart ein Verfahren zum Diagnostizieren einer Dynamik eines Abgassensors, mittels dem eine Eigenschaft eines Abgasstroms charakterisierbar ist, wobei der Abgassensor einen Messregelkreis aufweist und mittels eines Stellsignals des Messregelkreises die Eigenschaft des Abgasstroms charakterisierbar ist, mit:
- Erzeugen einer Änderung des Stellsignals ,
- Ermitteln einer Reaktion eines Messsignals des Messregelkreises auf die Änderung, und
- Beurteilen der Dynamik des Abgassensors mittels der Reaktion.

In der Schrift wird auf die Möglichkeit hingewiesen, dass anstatt einer Gemischzusammensetzung des Abgasmassenstroms durch Ab- und/ oder Umschalten des Reglers bzw. dessen Stellsignals, was beispielsweise in einem integrierten Schaltkreis des Reglers bzw. eines Motorsteuergerätes eines den Abgasstrom produzierenden Verbrennungsmotors geschehen kann, der Sauerstoffgehalt in der Referenzzelle geändert werden kann. Dabei wird nicht die Möglichkeit zur Diagnose der Fettmessfähigkeit des Abgassensors bei mager betriebenen Motoren offenbart.

Es ist daher Aufgabe der Erfindung, ein Verfahren und eine Vorrichtung bereitzustellen, welche es ermöglichen, eine Überprüfung der Funktionsfähigkeit einer Abgas-Sonde und eines zugehörigen Messkreises zu einer Bestimmung der Zusammensetzung eines fetten Abgas-Gemischs durchzuführen, ohne die Abgas-Sonde mit fettem Abgas zu beaufschlagen.

### Offenbarung der Erfindung

Die das Verfahren betreffende Aufgabe der Erfindung wird dadurch gelöst, dass während eines Magerbetriebs der Brennkraftmaschine in einer Diagnosephase die Pumprichtung der Pumpzelle umgekehrt und Sauerstoff in den Messhohlraum gepumpt wird und dass aus dem Pumpstrom und/oder der Pumpspannung auf die Fettgas-Messfähigkeit der Abgas-Sonde geschlossen wird.

Dabei kann es vorgesehen sein, dass auf eine intakte Abgas-Sonde oder eine intakte zugeordnete Sondenelektronik geschlossen wird, wenn sich eine zum Magerbetrieb umgekehrte Pumpstromrichtung einstellen lässt.

Im normalen Messbetrieb bei einer mager betriebenen Brennkraftmaschine, beispielsweise bei einem Dieselmotor, diffundiert Sauerstoff aus dem Abgas durch die Diffunsionsbarriere in den Messhohlraum der Abgas-Sonde. Entsprechend der Betriebsstrategie herrscht in dem Messhohlraum beispielsweise ein Lambda von 1. Der in den Messhohlraum eindiffundierende Sauerstoff wird mit der Pumpzelle mit einem positiven Pumpstrom abtransportiert.

Zur Diagnose der Fettgas-Messfähigkeit wird die Pumpspannung so modifiziert, dass sich ein negativer Pumpstrom einstellen muss. Dabei wird Sauerstoff in den Messhohlraum gepumpt. Für die Diagnose wird der gemessene Pumpstrom ausgewertet. Tritt kein negativer Pumpstrom auf, liegt ein Defekt der Abgas-Sonde oder des zugeordneten Messkreises vor.

Das Verfahren ermöglicht die Überprüfung der Fettgas-Messfähigkeit der Abgas-Sonde und der zugeordneten Sondenelektronik, ohne dass der Abgas-Sonde fettes Abgas zugeführt werden muss. Ein erhöhter Kraftstoffverbrauch sowie erhöhte Schadstoffemissionen während der Diagnose können so vermieden werden. Die Überwachung kann daher ausreichend oft durchgeführt werden, beispielsweise um die Anforderungen des "in use monitor performance ratio" (IUMPR) einzuhalten.

Eine genaue Bewertung der Fettgas-Messfähigkeit der Abgassonde kann dadurch erreicht werden, dass die Pumpspannung oder der Pumpstrom oder der zeitliche Verlauf der Pumpspannung oder der zeitliche Verlauf des Pumpstroms bei umgekehrter Pumpstromrichtung oder die Zusammensetzung des Abgases zur Bewertung der Fettgas-Messfähigkeit der Abgas-Sonde jeweils für sich betrachtet oder in Kombination der Kenngrößen berücksichtigt wird.

Entsprechend einer weiteren Variante der Erfindung kann es vorgesehen sein, dass mit Hilfe eines Reglers ein vorgegebener umgekehrter Pumpstrom eingestellt wird und dass zur Bewertung der Fettgas-Messfähigkeit der Abgas-Sonde die dazu notwendige Pumpspannung ausgewertet wird. Auch hierbei kann die Zusammensetzung des Abgases an der Abgas-Sonde mit berücksichtigt werden.

Eine weiterführende Diagnosemöglichkeit für die Abgas-Sonde ergibt sich aus der Möglichkeit, dass im Anschluss an die Diagnosephase in einem nachfolgenden Messbetrieb die Pumprichtung der Pumpzelle erneut umgekehrt und Sauerstoff aus dem Messhohlraum gepumpt wird und dass aus einem sich ergebenden maximalen Pumpstrom auf eine Sauerstoffanreicherung in dem Messhohlraum und/oder auf eine Leistungsfähigkeit der Pumpzelle und/oder auf eine verfügbare Funktionsreserve der Abgas-Sonde für den Magerbetrieb geschlossen wird.

Nach der Rückkehr von der Diagnosephase in den Messbetrieb ist das Signal der Abgas-Sonde für die Bestimmung der Abgaszusammensetzung für kurze Zeit ungültig, bis sich wieder näherungsweise ein Lambda von 1 in dem Messhohlraum eingestellt hat. Um eine Fehlmessung zu vermeiden kann es daher vorgesehen sein, dass nach einer Diagnosephase in dem regulären Messbetrieb eine Auswertung des Ausgangssignals der Abgas-Sonde nach einer vorgegebenen Wartezeit erfolgt.

Die die Vorrichtung betreffende Aufgabe der Erfindung wird dadurch gelöst, dass in der Steuereinheit ein Schaltkreis oder ein Programmablauf zur Umkehrung der Pumprichtung der Pumpzelle während eines Magerbetriebs der Brennkraftmaschine und während einer Diagnosephase der Abgas-Sonde vorgesehen ist, und dass die Steuereinheit einen Schaltkreis oder einen Programmablauf zur Diagnose einer korrekten Fettgas-Messfähigkeit bei Auftreten von negativen Stromwerten während der Diagnosephase enthält. Die Vorrichtung ermöglicht somit die Durchführung des beschriebenen Verfahrens.

Entsprechend einer besonders bevorzugten Ausgestaltungsvariante der Erfindung kann es vorgesehen sein, dass die Abgas-Sonde als Breitband-Lambdasonde oder als Stickoxid-Sensor ausgebildet ist. Bei beiden Abgas-Sonden wird die Konzentration der jeweiligen Abgaskomponente, also Sauerstoff oder Stickstoffoxyd (NOₓ), an Hand eines sich ausbildenden Pumpstroms ermittelt.

In einer weiteren Variante der Erfindung kann die Abgas-Sonde als Einzellen-Grenzstromsonde mit integriertem Sauerstoffspeicher ausgeführt sein.

### Kurze Beschreibung der Zeichnungen

Die Erfindung wird im Folgenden anhand eines in den Figuren dargestellten Ausführungsbeispiels näher erläutert. Es zeigt:
Figur 1 in schematischer Darstellung ein Sensorelement einer zweizelligen Breitband-Lambdasonde,
Figur 2 das Sensorelement einer einzelligen Abgas-Sonde.

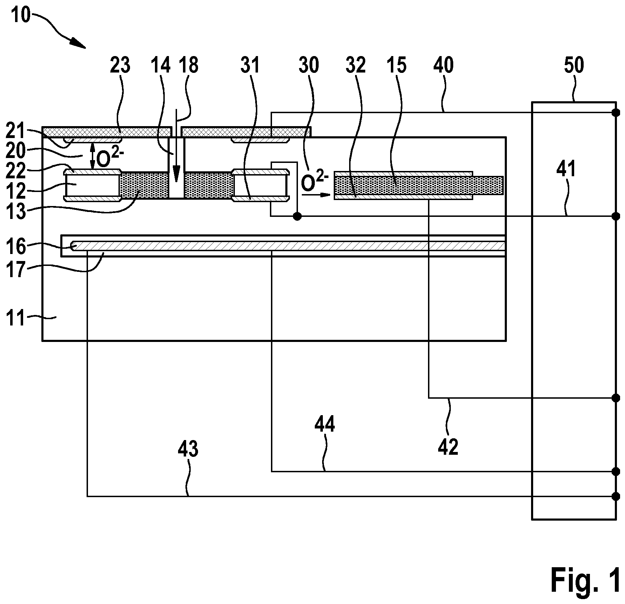

Figur 1 zeigt in schematischer Darstellung ein Sensorelement 10 einer planaren, zweizelligen Breitband-Lambdasonde zur Bestimmung eines Lambdawertes eines Abgases 18 mit einem Steckergehäuse 50. Das Sensorelement 10 ist im Wesentlichen aus einem Festkörperelektrolyten 11 aufgebaut. Es enthält eine Pumpzelle 20, eine Konzentrationszelle 30 und ein Heizelement 16. Der Festkörperelektrolyt 11 ist in der Darstellung als einheitlicher Körper aus Sauerstoffionen leitendem Zirkondioxid gezeigt, er kann jedoch im Aufbau aus einer Mehrzahl von Festelektrolytschichten aufgebaut sein.

Die Pumpzelle 20 besteht aus einer in einem Messhohlraum 12 angeordneten inneren Pumpelektrode 22 und einer äußeren zweiten Pumpelektrode 21. Die äußere Pumpelektrode 21 ist, abgedeckt durch eine Schutzschicht 23, dem Abgas 18 einer nicht dargestellten Brennkraftmaschine ausgesetzt. Die äußere Pumpelektrode 21 und die innere Pumpelektrode 22 sind ringförmig um einen Diffusionskanal 14 angeordnet. Der Diffusionskanal 14 führt das Abgas 18 über eine Diffusionsbarriere 13 dem Messhohlraum 12 zu.

Auf der der inneren Pumpelektrode 22 gegenüberliegenden Seite des Messhohlraums 12 ist eine Messelektrode 31 angeordnet. Die Messelektrode 31 bildet zusammen mit einer in einem Referenzkanal 15 angeordneten Referenzelektrode 32 und dem dazwischen liegenden Festkörperelektrolyten 11 die Konzentrationszelle 30. Der Referenzkanal 15 ist mit einem luftdurchlässigen Material gefüllt und zur Außenluft als Referenzgas geöffnet. Im Falle einer gepumpten Referenz kann der Referenzkanal 15 auch vollständig oder weitgehend geschlossen und beispielsweise mit Zirkonoxid gefüllt sein.

Das Heizelement 16 ist durch ein Isolationsmaterial 17 von dem Festkörperelektrolyten 11 elektrisch getrennt.

Die äußere Pumpelektrode 21 ist über einen Anschluss APE 40 mit dem Steckergehäuse 50 verbunden. Die innere Pumpelektrode 22 und die Messelektrode 31 sind parallel geschaltet und über einen gemeinsamen Anschluss IPN 41 zum Steckergehäuse 50 geführt. Die Referenzelektrode 32 ist über einen Anschluss RE 42 und das Heizelement 16 über einen ersten Heizeranschluss 43 und einen zweiten Heizeranschluss 44 an das Steckergehäuse 50 angeschlossen. Über das Steckergehäuse 50 erfolgt die elektrische Verbindung der Abgas-Sonde zu einer nicht dargestellten Sondenelektronik beziehungsweise Steuereinheit.

Im Betrieb der Breitband-Lambdasonde diffundiert Abgas 18 über den Diffusionskanal 14 und die Diffusionsbarriere 13 in den Messhohlraum 12. Über die Konzentrationszelle 30 wird der Lambdawert in dem Messhohlraum 12 durch Messung der Nernstspannung zwischen der Messelektrode 31 und der Referenzelektrode 32 bestimmt. Die Konzentrationszelle 30 ermöglicht dabei die Bestimmung des Lambdas in einem engen Messfenster um Lambda = 1. Durch Anlegen einer entsprechend polarisierten Spannung zwischen der zweiten und der inneren Pumpelektrode 21, 22 können Sauerstoffionen durch den Festkörperelektrolyten 11 von dem Abgas 18 in den Messhohlraum 12 oder aus dem Messhohlraum 12 zu dem Abgas 18 gepumpt werden.

Durch eine geeignete Regelung des zwischen den Pumpelektroden 21, 22 fließenden Pumpstroms und damit des Austausch von Sauerstoffionen zwischen dem Messhohlraum 12 und dem Abgas 18 wird das Lambda in dem Messhohlraum 12 auf einen Wert von 1 geregelt. Der Lambda-Wert in dem Messhohlraum 12 wird dabei durch die Konzentrationszelle 30 überwacht. Der Wert des dazu notwendigen Pumpstroms ist abhängig von der Sauerstoffkonzentration und somit von dem zu bestimmenden Lambdawert des Abgases 18 sowie von den Diffusionseigenschaften der Diffusionsbarriere 13.

Wird eine solche zweizellige Breitband-Lambdasonde im Abgas 18 eines mager betriebenen Dieselmotors eingesetzt, so diffundiert Sauerstoff aus dem Abgas 18 über den Diffusionskanal 14 und die Diffusionsbarriere 13 in den Messhohlraum 12. Der Sauerstoff wird durch eine entsprechend zwischen der inneren Pumpelektrode 22 und der äußeren zweiten Pumpelektrode 21 angelegten Spannung und dem daraus folgenden positiven Pumpstrom aus dem Messhohlraum 12 in das Abgas 18 gepumpt.

Im Rahmen einer On-Board-Diagnose wird die korrekte Funktion der Breitband-Lambdasonde überwacht. Dazu muss auch die Fettgas-Messfähigkeit der Breitband-Lambdasonde nachgewiesen werden. Im Falle eines Dieselmotors müsste dazu durch einen entsprechenden Eingriff in den Motorbetrieb ein fettes Abgas 18 eingestellt werden, was jedoch auf Grund des negativen Einflusses auf die Schadstoffemissionen und den Kraftstoffverbrauch nachteilig ist. Erfindungsgemäß ist es daher vorgesehen, während einer Diagnosephase einen negativen Pumpstrom mittels einer gesteuerten Veränderung der Pumpspannung einzustellen. In diesem Fall wird Sauerstoff an der inneren Pumpelektrode 22 in den Messhohlraum 12 abgegeben, sofern tatsächlich ein negativer Pumpstrom fließt. In dem Messhohlraum 12 bildet sich folglich ein Gas mit einem Lambda größer 1.

Für die Diagnose wird der gemessene Pumpstrom ausgewertet. Tritt kein negativer Pumpstrom auf, liegt ein Defekt vor. Darüber hinaus kann unter Berücksichtigung der Gaszusammensetzung an dem Sensorelement 10 auch die Pumpspannung ausgewertet werden, die an der Pumpzelle 20 eingestellt wird. In einer weiteren Ausbaustufe ist ein Regler vorgesehen, welcher versucht, einen vorgegebenen negativen Pumpstrom einzustellen. Hierbei kann die dafür notwendige Pumpspannung ausgewertet werden, ebenfalls unter Berücksichtigung der Zusammensetzung des vorliegenden Abgases 18.

Das Verfahren ermöglicht es beispielsweise, eine Manipulation der Breitband-Lambdasonde und der angeschlossenen Sondenelektronik, bei welcher eine Diode in den Pumpzellenkreis zur Verhinderung eines negativen Pumpstroms eingebracht wurde, zu erkennen.

Nach der Diagnosephase zu Beginn des nachfolgenden Messbetriebs ist das Signal der Breitband-Lambdasonde noch für eine kurze Zeit für die Bestimmung der Zusammensetzung des Abgases 18 ungültig, bis sich wieder ein Lambda von 1 in dem Messhohlraum 12 eingestellt hat.

In einer weiteren Ausbaustufe der Erfindung wird der erhöhte Pumpstrom, der sich bei der Rückkehr in den regulären Messbetrieb durch das Leerpumpen des Messhohlraums 12 einstellt, ausgewertet. Der Peak des Pumpstroms ist sowohl ein Maß für die Sauerstoffanreicherung in dem Messhohlraum 12 als auch ein Maß für die Leistungsfähigkeit der Pumpzelle 20. Er eignet sich weiterhin für die Diagnose der verfügbaren Funktionsreserve für den Magerbetrieb der Breitband-Lambdasonde.

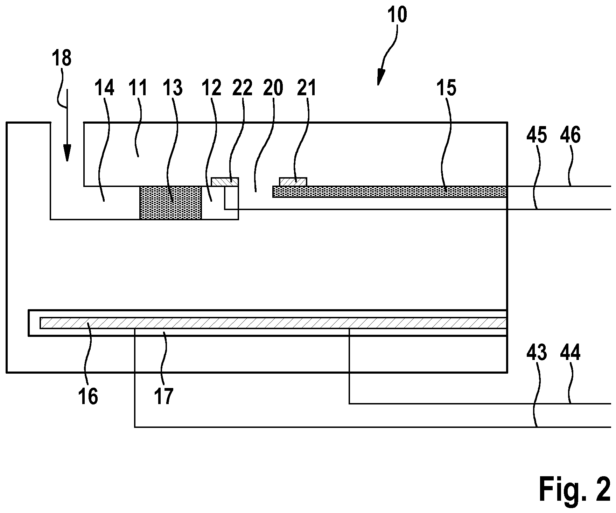

Figur 2 zeigt das Sensorelement 10 einer einzelligen Abgas-Sonde mit einem Festkörper-Elektrolyten 11 als Grundkörper und einem Diffusionskanal 14, der zu einem Abgaskanal einer nicht dargestellten Brennkraftmaschine hin geöffnet ist. Die Abgas-Sonde dient als Lambda-Sonde zur Bestimmung der Zusammensetzung des der Brennkraftmaschine zugeführten Luft-Kraftstoff-Gemischs. Der Festkörper-Elektrolyt 11 ist beispielhaft aus Yttrium-stabilisiertem Zirkoniumdioxid gefertigt, das bei Betriebstemperatur ionenleitend ist und mit einer geeigneten Anordnung von Elektroden und Gas-Zuführungen zur Bestimmung einer Konzentration bestimmter Komponenten einer Gas-Zusammensetzung geeignet ist. Abgas 18 tritt über den Diffusionskanal 14 durch eine Diffusionsbarriere 13 in einen Messhohlraum 12. Die Diffusionsbarriere 13 ist ein poröses Element, welches ein Nachströmen von Gas aus dem Diffusionskanal 14 in den Messhohlraum 12 oder in umgekehrter Richtung zumindest weitgehend verhindert und lediglich einen Diffusionstransport ermöglicht. In dem Messhohlraum 12 ist ein Teil der Wandung des Festkörper-Elektrolyten 11 mit einer ersten inneren Pumpelektrode 22 belegt, die über eine zweite Zuleitung 46 nach außen geführt ist. Der Festkörper-Elektrolyt 11 weist weiterhin einen Referenzgaskanal 15 auf, der mit einem porösen, gasdurchlässigen Medium ausgefüllt ist und dessen Wandung teilweise mit einer zweiten Pumpelektrode 21 belegt ist. Die zweite Pumpelektrode 21 ist über eine erste Zuleitung 45 nach außen geführt. Der Referenzgaskanal 15 wird auch als Abluftkanal (ALK), die zweite Pumpelektrode 21 auch als Abluftelektrode (ALE) bezeichnet. Die innere Pumpelektrode 22 und die zweite Pumpelektrode 21 sind in dem dargestellten Ausführungsbeispiel im Inneren des als Schichtaufbau ausgeführten Grundkörpers angeordnet. Die innere Pumpelektrode 22, die zweite Pumpelektrode 21 und der dazwischen liegende Teil des Festkörper-Elektrolyten 11 bilden gemeinsam eine Pumpzelle 20.

Weiterhin umfasst die Abgas-Sonde in dem dargestellten Ausführungsbeispiel ein Heizelement 16 mit einem Isolationsmaterial 17, welche das Heizelement 16 umgibt und bei Betriebstemperatur einen elektrischen Kontakt zu anderen Komponenten in der Abgas-Sonde verhindert. Das Heizelement 16 wird über einen ersten Heizanschluss 43 und einen zweiten Heizanschluss 44 mit Betriebsspannung versorgt. Die Betriebsspannung wird von einer zugeordneten Steuerung oder Sondenelektronik derart geregelt, dass sich ein vorgegebener Innenwiderstand der Pumpzelle 20 einstellt. In der Steuerung beziehungsweise Sondenelektronik werden auch die elektrischen Spannungen an der inneren Pumpelektrode 22 und zweiten Pumpelektrode 21 vorgegeben oder bestimmt und die Ströme in der ersten und zweiten Zuleitung 45, 46 vorgegeben oder bestimmt. Die innere Pumpelektrode 22, welche mit Abgas 18 beaufschlagt ist, ist als Beispiel einer Realisierung an eine virtuelle Masse der Steuerung angeschlossen. Diese virtuelle Masse legt die erste innere Pumpelektrode 22 auf ein konstantes Elektrodenpotenzial relativ zu einer elektrischen Masse. Die zweite Pumpelektrode 17 oder Abluftelektrode hingegen liegt auf einem variablen Potenzial. Über eine Pumpspannungsquelle wird mittels einer Strommessvorrichtung, beispielhaft mittels eines Messwiderstands, ein Pumpstrom I_{P} durch die Pumpzelle 20 bestimmt. Bei üblichen Schaltungen erfolgt dies derart, dass über eine Einspeisung an einem nichtinvertierenden Eingang eines Operationsverstärkers eine Pumpspannung U_{P} der Pumpspannungsquelle geregelt wird, um bei einer Messung an Luft eine höhere Pumpspannung von 900 mV, bei Fettgas hingegen eine kleinere Pumpspannung U_{P} von 200 mV einzustellen. Zwischen der inneren Pumpelektrode 22 und der zweiten Pumpelektrode 21 stellt sich eine von der Zusammensetzung des Abgases abhängige Nernst-Spannung U_{N} ein.

Wie bei der in Figur 1 gezeigten zweizelligen Breitband-Lambdasonde diffundiert auch bei der hier gezeigten einzelligen Abgas-Sonde bei einem mageren Abgas 18 Sauerstoff aus dem Abgas 18 über den Diffusionskanal 14 und die Diffusionsbarriere 13 in den Messhohlraum 12. Der Sauerstoff wird durch eine entsprechend zwischen der inneren Pumpelektrode 22 und der zweiten Pumpelektrode 21 angelegten Spannung und dem daraus folgenden positiven Pumpstrom aus dem Messhohlraum 12 in den Referenzkanal 15 gepumpt.

Erfindungsgemäß wird zur Diagnose der Fettmessfähigkeit der Abgas-Sonde bei weiterhin magerem Abgas 18 vorübergehend eine Pumpspannung U_{P} zwischen der inneren Pumpelektrode 22 und der zweiten Pumpelektrode 21 angelegt, welche einen negativen Pumpstrom ergibt. Hierdurch wird Sauerstoff aus dem Referenzgaskanal 15 in den Messhohlraum 12 gepumpt. Während dieser Diagnosephase ist das AusgangsSignal der Abgas-Sonde nicht zur Bestimmung des Lambdawerts geeignet.

Für die Diagnose wird, wie bereits zu Figur 1 beschrieben, der gemessene Pumpstrom ausgewertet. Tritt kein negativer Pumpstrom auf, liegt ein Defekt vor. Dabei können bei der einzelligen Abgas-Sonde die gleichen Auswerteverfahren wie bei der in Figur 1 beschriebenen zweizelligen Breitband-Lambdasonde angewendet werden.

Die Diagnose auch bei digitalem, diskontinuierlichem Betrieb der elektrochemischen Zelle mit einem pulsierenden Pumpstrom möglich.

## Patentansprüche

1. Verfahren zur Überwachung einer Fettgas-Messfähigkeit einer Abgas-Sonde in einem Abgaskanal einer mager betriebenen Brennkraftmaschine, wobei in der Abgas-Sonde Abgas (18) über eine Diffusionsbarriere (13) von dem Abgaskanal in einen Messhohlraum (12) der Abgas-Sonde diffundiert und wobei über eine Pumpzelle (20) mit einer inneren Pumpelektrode (22) und einer zweiten Pumpelektrode (21) durch Anlegen einer Pumpspannung zwischen den Elektroden (21, 22) Sauerstoff entsprechend eines fließenden Pumpstroms in den oder aus dem Messhohlraum (12) gepumpt wird, **dadurch gekennzeichnet, dass** während eines Magerbetriebs der Brennkraftmaschine in einer Diagnosephase die Pumprichtung der Pumpzelle (20) umgekehrt und Sauerstoff in den Messhohlraum (12) gepumpt wird und dass aus dem Pumpstrom und/oder der Pumpspannung auf die Fettgas-Messfähigkeit der Abgas-Sonde geschlossen wird.

2. Verfahren nach Anspruch 1, **dadurch gekennzeichnet, dass** auf eine intakte Abgas-Sonde oder eine intakte zugeordnete Sondenelektronik geschlossen wird, wenn sich eine zum Magerbetrieb umgekehrte Pumpstromrichtung einstellen lässt.

3. Verfahren nach Anspruch 1 oder 2, **dadurch gekennzeichnet, dass** die Pumpspannung oder der Pumpstrom oder der zeitliche Verlauf der Pumpspannung oder der zeitliche Verlauf des Pumpstroms bei umgekehrter Pumpstromrichtung oder die Zusammensetzung des Abgases (18) zur Bewertung der Fettgas-Messfähigkeit der Abgas-Sonde jeweils für sich betrachtet oder in Kombination der Kenngrößen berücksichtigt wird.

4. Verfahren nach einem der Ansprüche 1 bis 3, **dadurch gekennzeichnet, dass** mit Hilfe eines Reglers ein vorgegebener umgekehrter Pumpstrom eingestellt wird und dass zur Bewertung der Fettgas-Messfähigkeit der Abgas-Sonde die dazu notwendige Pumpspannung ausgewertet wird.

5. Verfahren nach einem der Ansprüche 1 bis 4, **dadurch gekennzeichnet, dass** im Anschluss an die Diagnosephase in einem nachfolgenden Messbetrieb die Pumprichtung der Pumpzelle (20) erneut umgekehrt und Sauerstoff aus dem Messhohlraum (12) gepumpt wird und dass aus einem sich ergebenden maximalen Pumpstrom auf eine Sauerstoffanreicherung in dem Messhohlraum (12) und/oder auf eine Leistungsfähigkeit der Pumpzelle (20) und/oder auf eine verfügbare Funktionsreserve der Abgas-Sonde für den Magerbetrieb geschlossen wird.

6. Verfahren nach einem der Ansprüche 1 bis 5, **dadurch gekennzeichnet, dass** nach einer Diagnosephase in dem regulären Messbetrieb eine Auswertung des Ausgangssignals der Abgas-Sonde nach einer vorgegebenen Wartezeit erfolgt.

7. Vorrichtung zur Überwachung einer Fettgas-Messfähigkeit einer Abgas-Sonde in einem Abgaskanal einer mager betriebenen Brennkraftmaschine, wobei ein Messhohlraum (12) der Abgas-Sonde über eine Diffusionsbarriere (13) mit dem Abgaskanal verbunden ist, wobei eine Pumpzelle (20) mit einer inneren Pumpelektrode (22) und einer zweiten Pumpelektrode (21) zum Pumpen von Sauerstoff in oder aus dem Messhohlraum (12) vorgesehen ist, wobei zum Auspumpen von Sauerstoff aus dem Messhohlraum (12) ein positiver und zum Einpumpen ein negativer Pumpstrom zwischen den Elektroden (21, 22) vorgesehen ist und wobei der Abgas-Sonde eine Steuereinheit zugeordnet ist, **dadurch gekennzeichnet, dass** in der Steuereinheit ein Schaltkreis oder ein Programmablauf zur Umkehrung der Pumprichtung der Pumpzelle (20) während eines Magerbetriebs der Brennkraftmaschine und während einer Diagnosephase der Abgas-Sonde vorgesehen ist, und dass die Steuereinheit einen Schaltkreis oder einen Programmablauf zur Diagnose einer korrekten Fettgas-Messfähigkeit bei Auftreten von negativen Stromwerten während der Diagnosephase enthält.

8. Vorrichtung nach Anspruch 7, **dadurch gekennzeichnet, dass** die Abgas-Sonde als Breitband-Lambdasonde oder als Stickoxid-Sensor ausgebildet ist.

9. Vorrichtung nach Anspruch 7 oder 8, **dadurch gekennzeichnet, dass** die Abgas-Sonde als Einzellen-Grenzstromsonde mit integriertem Sauerstoffspeicher ausgeführt ist.

## Claims

1. Method for monitoring the capability of an exhaust gas probe to measure a fuel rich mixture in an exhaust gas tract of a lean-operated internal combustion engine, wherein, in the exhaust gas probe, exhaust gas (18) diffuses from the exhaust gas tract into a measuring cavity (12) in the exhaust gas probe via a diffusion barrier (13), and wherein oxygen is pumped into or out of the measuring cavity (12) in accordance with a flowing pump current via a pump cell (20) having an inner pump electrode (22) and a second pump electrode (21), by applying a pump voltage between the electrodes (21, 22), **characterized in that** during lean operation of the internal combustion engine, in a diagnostic phase the pumping direction of the pump cell (20) is reversed and oxygen is pumped into the measuring cavity (12), and **in that** the capability of the exhaust gas probe to measure a fuel rich mixture is concluded from the pump current and/or the pump voltage.

2. Method according to Claim 1, **characterized in that** it is concluded that there is an intact exhaust gas probe or intact associated probe electronics if a reversed pump current direction can be set for lean operation.

3. Method according to Claim 1 or 2, **characterized in that** in order to assess the capability of the exhaust gas probe to measure a fuel rich mixture, the pump voltage or the pump current or the time profile of the pump voltage or the time profile of the pump current with the pump current direction reversed or the composition of the exhaust gas (18) is respectively taken into account, on its own or in combination of the characteristic variables.

4. Method according to one of Claims 1 to 3, **characterized in that** a predefined reversed pump current is set with the aid of a controller, and **in that** the pump voltage needed for this purpose is evaluated to assess the capability of the exhaust gas probe to measure the fuel rich mixture.

5. Method according to one of Claims 1 to 4, **characterized in that**, following the diagnostic phase, in a subsequent measurement operation the pumping direction of the pump cell (20) is again reversed and oxygen is pumped out of the measuring cavity (12), and **in that** conclusions are drawn from a resulting maximum pump current about oxygen enrichment in the measuring cavity (12) and/or about serviceability of the pump cell (20) and/or about an available functional reserve of the exhaust gas probe for lean operation.

6. Method according to one of Claims 1 to 5, **characterized in that** after a diagnostic phase, during regular measuring operation an evaluation of the output signal from the exhaust gas probe is carried out after a predefined waiting time.

7. Device for monitoring the capability of an exhaust gas probe to measure a fuel rich mixture in an exhaust gas tract of a lean operated internal combustion engine, a measuring cavity (12) of the exhaust gas probe being connected to the exhaust gas tract via a diffusion barrier (13), a pump cell (20) having an inner pump electrode (22) and a second pump electrode (21) being provided to pump oxygen into or out of the measuring cavity (12), a positive pump current being provided between the electrodes (21, 22) to pump oxygen out of the measuring cavity (12) and a negative pump current being provided to pump oxygen in, and the exhaust gas probe being assigned a control unit, **characterized in that** a circuit or program sequence for reversing the pumping direction of the pump cell (20) during lean operation of the internal combustion engine and during a diagnostic phase of the exhaust gas probe is provided in the control unit, and **in that** the control unit contains a circuit or a program sequence to diagnose a correct capability of measuring a fuel rich mixture if negative current values occur during the diagnostic phase.

8. Device according to Claim 7, **characterized in that** the exhaust gas probe is formed as a broadband Lambda probe or as a nitrogen oxide sensor.

9. Device according to Claim 7 or 8, **characterized in that** the exhaust gas probe is implemented as a single-cell limiting-current probe with integrated oxygen reservoir.

## Revendications

1. Procédé pour surveiller une aptitude au mesurage du gaz gras d'une sonde de gaz d'échappement dans un canal de gaz d'échappement d'un moteur à combustion interne à combustion maigre, le gaz d'échappement (18) diffusant dans la sonde de gaz d'échappement via une barrière de diffusion (13) depuis le canal de gaz d'échappement dans un espace creux (12) de mesure de la sonde de gaz d'échappement et de l'oxygène étant pompé conformément à un courant de pompage qui s'écoule dans ou hors de l'espace creux (12) de mesure via une cellule de pompage (20) présentant une électrode interne (22) de pompage et une deuxième électrode (21) de pompage par application d'une tension de pompage entre les électrodes (21, 22), **caractérisé en ce que**, pendant la combustion maigre du moteur à combustion interne, dans une phase de diagnostic, le sens de pompage de la cellule de pompage (20) est inversé et l'oxygène est pompé dans l'espace creux (12) de mesure et **en ce que** l'aptitude au mesurage du gaz gras de la sonde de gaz d'échappement est déduite du courant de pompage et/ou de la tension de pompage.

2. Procédé selon la revendication 1, **caractérisé en ce qu'**on déduit que la sonde de gaz d'échappement est intacte ou que l'électronique associée à la sonde est intacte lorsqu'on peut régler une direction de courant de pompage inversée par rapport à la combustion maigre.

3. Procédé selon la revendication 1 ou 2, **caractérisé en ce que** la tension de pompage ou le courant de pompage ou l'allure temporelle de la tension de pompage ou l'allure temporelle du courant de pompage lors de la direction inversée du courant de pompage ou la composition du gaz d'échappement (18) sont prises en considération, respectivement en soi ou en combinaison des caractéristiques, pour évaluer l'aptitude au mesurage du gaz gras de la sonde de gaz d'échappement.

4. Procédé selon l'une quelconque des revendications 1 à 3, **caractérisé en ce qu'**un courant de pompage inversé prédéfini est réglé à l'aide d'un régulateur et **en ce que** la tension de pompage nécessaire à cet effet est évaluée pour l'évaluation de l'aptitude au mesurage du gaz gras de la sonde de gaz d'échappement.

5. Procédé selon l'une quelconque des revendications 1 à 4, **caractérisé en ce que**, à la suite de la phase de diagnostic, dans un fonctionnement de mesurage consécutif, le sens de pompage de la cellule de pompage (20) est de nouveau inversé et de l'oxygène est pompé hors de l'espace creux (12) de mesurage et **en ce que** qu'on déduit un enrichissement en oxygène dans l'espace creux (12) de mesurage et/ou une performance de la cellule de pompage (20) et/ou une réserve de fonctionnement disponible de la sonde de gaz d'échappement pour le fonctionnement maigre à partir du courant de pompage maximal obtenu.

6. Procédé selon l'une quelconque des revendications 1 à 5, **caractérisé en ce qu'**on effectue une évaluation du signal de départ de la sonde de gaz d'échappement après un temps d'attente prédéfini après une phase de diagnostic dans le fonctionnement de mesurage normal.

7. Dispositif pour surveiller une aptitude au mesurage du gaz gras d'une sonde de gaz d'échappement dans un canal de gaz d'échappement d'un moteur à combustion interne à combustion maigre, un espace creux (12) de mesurage de la sonde de gaz d'échappement étant relié via une barrière de diffusion (13) au canal de gaz d'échappement, une cellule de pompage (20) présentant une électrode interne (22) de pompage et une deuxième électrode (21) de pompage étant destinée au pompage d'oxygène dans ou hors de l'espace creux (12) de mesure, un courant de pompage positif pour le pompage de l'oxygène hors de l'espace creux (12) de mesurage et un courant de pompage négatif pour le pompage de l'oxygène dans l'espace creux de mesurage étant utilisés entre les électrodes (21, 22) et une unité de commande étant associée à la sonde de gaz d'échappement, **caractérisé en ce que** l'unité de commande présente un circuit de commutation ou un déroulement de programme pour inverser le sens de pompage de la cellule de pompage (20) pendant une combustion maigre du moteur à combustion interne et pendant une phase de diagnostic de la sonde de gaz d'échappement et **en ce que** l'unité de commande présente un circuit de commutation ou un déroulement de programme pour le diagnostic d'une aptitude au mesurage correcte du gaz gras lors de l'apparition de valeurs négatives de courant pendant la phase de diagnostic.

8. Dispositif selon la revendication 7, **caractérisé en ce que** la sonde de gaz d'échappement est conçue en tant que sonde lambda à large bande ou en tant que sonde à oxyde d'azote.

9. Dispositif selon la revendication 7 ou 8, **caractérisé en ce que** la sonde de gaz d'échappement est réalisée sous forme de sonde de courant limite à cellule unique présentant un accumulateur d'oxygène intégré.
